# EUROPEAN PATENT SPECIFICATION

(11) **EP 1 890 144 B1**
(45) Date of publication and mention of the grant of the patent: **15.12.2010**
(21) Application number: 07380235.7
(22) Date of filing: 09.08.2007
(51) Int. Cl.: G01N 33/50, B01L 99/00, B65D 5/20

(54) **Portable biotechnology kit**
Tragbares Biotechnologie kit
kit de biotechnologique portable

(30) Priority: 11.08.2006 ES 200700311
(43) Date of publication of application: 20.02.2008
(73) Proprietor: UNIVERSIDAD DEL PAIS VASCO-EUSKAL HERRIKO UNIBERSITATEA, 48940 Leioa (Vizcaya) (ES); Laboratorios aidelos SRL, 48150 Sondika (Vizcaya) (ES)
(72) Inventor: Martinez de Pancorbo, 48993 Getxo (Vizcaya) (ES); Rosique Sola, Melania Julieta, 48620 Plentzia (Vizcaya) (ES); Ramos de Pablo, Rodrigo, 01009 Vitoria-Gasteiz (Alava) (ES); Eguskiza Madariaga, Gaizka, 48630 Gorliz (Vizcaya) (ES)
(74) Representative: Carpintero Lopez, Francisco

(56) References cited:
- WO-A-99/16675
- CA-A1- 2 268 204
- DE-A1- 19 812 649
- ANONYMOUS: "BIOTECHNOLOGY EXPLORER -Captivating Science Education" CATALOGUE BIO-RAD, January 2006 (2006-01), XP002477912
- ANONYMOUS: "Program for general microbiology lesson (BY44)" SUFFOLK COUNTY COMMUNITY COLLEGE EASTERN CAMPUS PROGRAMS, [Online] March 2006 (2006-03), XP002476810 Riverhead, New York Retrieved from the Internet: URL:http://www.sunysuffolk.edu/Web/East/RM FS/East%20Outlines/outlines/by44_czura_a.p df> [retrieved on 2008-04-14]
- ANONYMOUS: "EDVO-Kit # 300 - Blue/White Cloning of a DNA Fragment and Assay of beta-Galactosidase" E D V O T E K EXPERIMENTS, [Online] XP002476809 Retrieved from the Internet: URL:http://www.edvotek.com/pdf/300.pdf> [retrieved on 2008-04-14]

## Description

### OBJECT OF THE INVENTION

The object of the present application is a portable kit for performing biotechnology laboratory practical lessons, allowing these to be performed in a simple manner and at low cost. Its main application is for educational purposes, although it may also be used for "on site" sample analyses, i.e. outside the laboratory.

### BACKGROUND OF THE INVENTION

The main problem for performing Biotechnology practicals in the Classroom is the availability of biological material equipment to obtain the intermediate product necessary for performing such practices. On the other hand, Biotechnology requires reagents, highly specialised consumables and protocols as well as practical experience in order to obtain results in a laboratory. All this entails great difficulty for the development of practicals by the teachers, a problem that has been solved by the development of the kits object of the present invention.

Portable kits for performing Biotechnology practicum are known in the art, for example the comparative Proteomics Kit by Bio-Rad, where sufficient material for the practicum, namely reagents and consumables, are provided in a regular box.

No other similar approaches are known to solving the problem of providing Biotechnology practical laboratory lessons.

### DESCRIPTION OF THE INVENTION

The present invention has as an object portable kits for performing biotechnology laboratory practical lessons. Each kit comprises at least one group box containing all the material necessary for the students to perform an entire specific biotechnology practical lesson. One of the most striking peculiarities of the invention is that the box is made from a flat sheet of cardboard, with folding lines that allow it to be turned into a closed container in which to transport all the material necessary for the practical, but which, once open, remains unfolded in a flat surface which can be used as a work surface. The box thus has adhesives that allow it to be fixed upon a table or support surface. The box, once the practical is finished, is folded again in order to form a disposable waste basket, including all the material used in the practical. The group box has a lid that keeps the box in its folded position, without the need for adhesives or other fastening means.

At least one group box includes different elements depending on the type of biotechnology practical and comprises:
at least one test-tube rack, of disposable material, for holding tubes, vials or Eppendorf tubes with samples, culture mediums or reagents.
At least one pipette.
At least one of the following elements: a micro vial, an Eppendorf tube, an agarose gel, an electrophoresis clamp, absorbent cellulose paper for cleaning the electrophoresis clamp, a small square of blotting paper in order to load an agarose gel, a sterile inoculation loop, a safety tube.

Kit configurations can be variable. Namely, the kit described as an example in the present application, is supplied in a box containing four group boxes. Inside the box are a Teacher's Manual and four other manuals for the four students who will perform the practical. Each box has a label indicating the name of the kit and the number of each group. The material inside it is labelled indicating the optimal preservation temperature to keep the reagents at until the practical is performed.

10 kits have been developed, which allow performing practicals for:
- Microbial Biotechnology.
- Molecular Biology.

Within those for Molecular Biology, specifically, we have created kits for "Bacterial DNA Extraction", "Diagnosis of Maternal Kinship", "Screenings for Transgenics" and "Identification of Meat Species of Interest in Nutrition". Within those for Microbial Biotechnology, we have created kits for "Bioproduction of Amylases and Proteases", "Bioproduction of Cellulases and β-Galactosidase", "Food Biotechnology", "Bacterial Differentiation", "Oxygen Tolerance" and "Mobility and Chemotaxis".

For each and every one of these kits we have chosen the techniques and components for each kit so that the result is an intermediate product for its easy transformation into the final result in the Classroom, in a simple manner and with great educational value. They thus allow the introduction of students, with or without experience, in handling molecular and diagnostic techniques, eliminating the phase of prior preparation for developing the practical lesson, and also eliminating preparation time by the teachers, who, on the other hand, do not require specific knowledge in Biotechnology.

In short, the invention provides the following advantages:
- The Biotechnology Kits are an effective means to integrate complex knowledge and simplify its assimilation.
- They are suited to the Biology syllabus and to class duration.
- The practical lessons are previously tested and the desired results are therefore guaranteed.
- They allow incorporating changes in practical protocols, reducing the risk of exposure to microorganisms for the students and ensuring the success of each practical.
- The kits are the result of the specific development of techniques for teaching practices which favour performing the practices and interpreting the results more easily.
- These are specific kits that allow performing experiments that are carried out in clinical and research laboratories inside the Classroom, broadening the range of experiments that can be performed in the Classroom with educational purposes.
- The results are easy to visualize, using microorganisms of low or no pathogenicity that are not included in biological risk groups 2, 3 or 4, according to Royal Decree 664/1997 of 12 May.
- They respect the environment, due to a new format design that allows reducing the generation of toxic and biological waste.

### BRIEF DESCRIPTION OF THE DRAWINGS

In order to complete this description and such as to aid towards a better understanding of the features of the invention according to a preferred embodiment thereof, a set of drawings is included as an integral part of said description representing in an illustrative and non-limiting manner the following:
Fig. 1 shows a box for a Kit or Kits.
Fig. 2 shows a box for a specific Kit.
Fig. 3 shows a box with a complete Kit with a set of Kits.
Fig. 4 shows a box for a specific Kit unfolded on a table, before being attached by means of the adhesives in the Kit box.
Fig. 5 shows the Kit completely unfolded and ready for use.

### DESCRIPTION OF A PREFERRED EMBODIMENT OF THE INVENTION

The kits are designed to carry out the activities in two practical sessions, and contain all the material for the students to distribute in four different groups, in order to carry out the practical lessons. They also allow dividing the kit into 1, 2 or 3 group boxes in order to perform the practicals in centres with few students.

Each kit is made up of:
- 1 Teacher's Manual.
- 4 Student Manuals.
- 4 group boxes with the consumables and reagents necessary for developing the practical by dividing the class into 4 groups. Said material is made up of test tubes, sterile inoculation loops, reagents, microorganism solutions, DNA samples, etc.

The materials will vary according to the different kits offered.

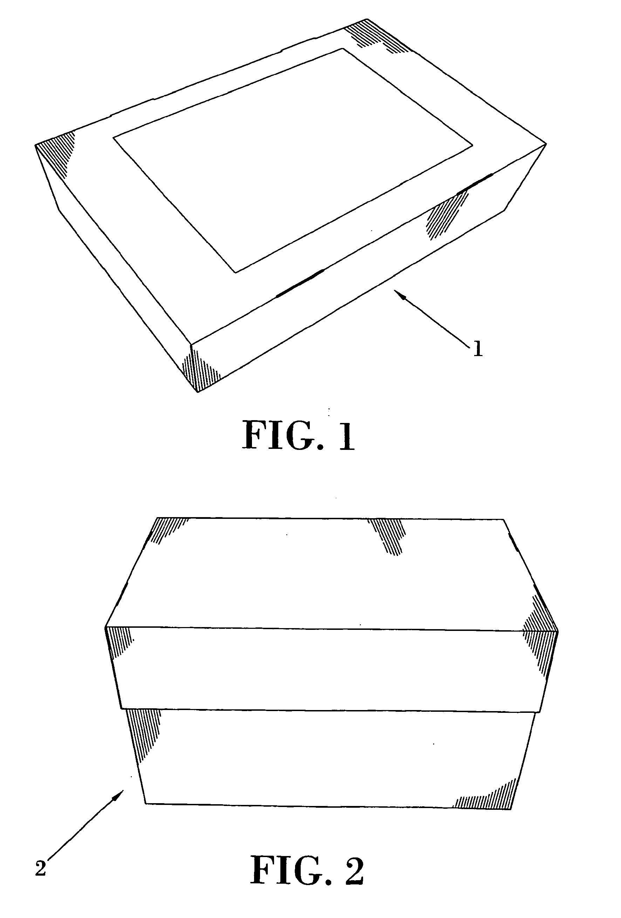

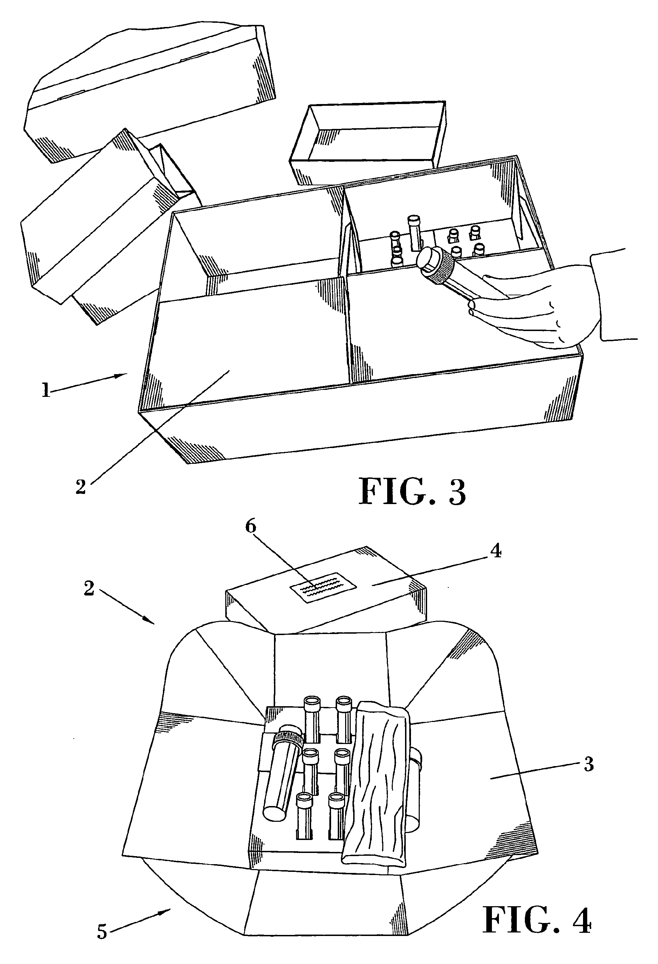

One of the kits will now be described as an example with reference to the figures. Namely, in Fig. 1 we can see a box (1) that can contain an individual Kit or a set of Kits, whereas Fig. 2 shows a box (2) with a specific Kit, namely the Kit for the "Screenings for Transgenics" practical. Fig. 3 shows the box of a group of Kits (1) and its contents, i.e. the specific Kits (2). Fig. 4 shows the box (2) of the "Screenings for Transgenics" Kit unfolded on a table, before being attached to said table with the adhesives provided in the Kit box, and showing its contents. More specifically, it shows the entire Kit (2), made up of the base of the box (3) unfolded and before being attached to the work table with the adhesives (5) and the lid (4) with the Kit's identification label (6). Inside the box we can see the test-tube rack (7)...

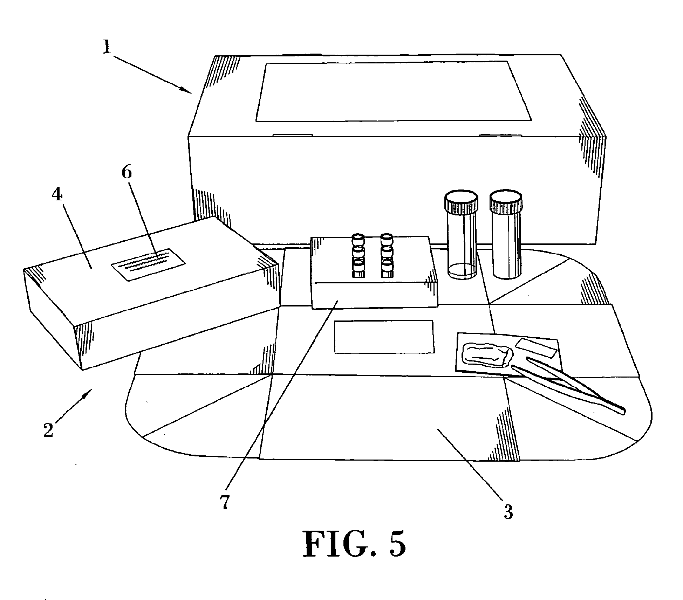

Fig. 5 shows the entire set ready for use.

We will now describe the contents of each one of the kits.

### MOLECULAR BIOLOGY

| MATERIALS | COMPONENTS PER GROUP |
|---|---|
| Sterile Pasteur Pipettes | • 1 pipette A for microorganism inoculation |
| Pasteur Pipettes for reagents | • 1 L1 pipette |
| | • 1 L2 pipette |
| Safety tube (store between 2-8 °C) | 1 vial with microorganisms |
| | • Vial A: *Citrobacter freundii* |
| Test-tube rack (store in the freezer) | • 1 L1 Eppendorf tube: Lysis solution 1 |
| | •1 Falcon P tube: DNA precipitation solution |
| Test-tube rack (store at the indicated temperature) | • 1 L2 Eppendorf tube: lysis solution 2 |
| | • 1 Falcon E tube: culture medium |

### DIAGNOSIS OF MATERNAL KINSHIP.

| MATERIALS | COMPONENTS PER GROUP |
|---|---|
| Test-tube rack (store between 2-8 °C) | • 1M1 Eppendorf tube: mtDNA of supposed mother 1 |
| | • 1 M2 Eppendorf tube: mtDNA of supposed mother 2 |
| | • 1 H Eppendorf tube: mtDNA of the son |
| | • 1 DNA(+) Eppendorf tube: Positive control with two mtDNA fragments of different sizes |
| | • 1 mtDNA(-) Eppendorf tube: Negative control |
| COMMON MATERIAL (included in each group box 1) | COMPONENTS PER KIT |
| Jar B (store between 2-8 °C) | • 1 Jar B: electrophoresis solution |
| Ethidium bromide (store at room temperature and in the dark) | • 1 BE Eppendorf tube: ethidium bromide |
| Pasteur Pipettes for reagents | • 1 BE pipette |
| Electrophoresis gels (store between 2-8 °C) | • 4 agarose gels (one for each group) with prepared wells |
| For gel loading | • 1 bag of small squares of blotting paper for gel loading |
| | • 1 clamp |
| | • 4 absorbent cellulose papers to clean the clamps |

### SCREENINGS FOR TRANSGENICS.

| COMMON MATERIAL (included in each group box 1) | COMPONENTS PER KIT |
|---|---|
| Jar B | • 1 Jar B: electrophoresis solution |
| Ethidium bromide (store at room temperature) | • 1 BE Eppendorf tube: ethidium bromide |
| Pasteur Pipettes for reagents | • 1 BE pipette |
| Electrophoresis gels (store between 2-8 °C) | • 4 agarose gels (one for each group) with prepared wells |
| For gel loading | • 1 bag of small squares of blotting paper for gel loading |
| | • 1 clamp |
| | • 4 absorbent cellulose papers to clean the clamps |

| MATERIALS | COMPONENTS PER GROUP |
|---|---|
| Test-tube rack group 1 (store between 2-8 °C) | • 1 A1 Eppendorf tube: DNA extracted from normal soybean with an amplified fragment of the lectin gene |
| | • 1 B1 Eppendorf tube: DNA extracted from normal soybean with an amplified fragment of the zein gene |
| | • 1 C1 Eppendorf tube: DNA extracted from normal soybean with an amplified fragment of the 35S gene |
| | • 1 D1 Eppendorf tube: DNA extracted from normal soybean with an amplified fragment of the NOS gene |
| Test-tube rack group 2 (store between 2-8 °C) | • 1 A2 Eppendorf tube: DNA extracted from transgenic soybean with an amplified fragment of the lectin gene |
| | • 1 B2 Eppendorf tube: DNA extracted from transgenic soybean with an amplified fragment of the zein gene |
| | • 1 C2 Eppendorf tube: DNA extracted from transgenic soybean with an amplified fragment of the 35S gene |
| | • 1 D2 Eppendorf tube: DNA extracted from transgenic soybean with an amplified fragment of the NOS gene |
| Test-tube rack group 3 (store between 2-8 °C) | • 1 A3 Eppendorf tube: DNA extracted from normal corn with an amplified fragment of the lectin gene |
| | • 1 B3 Eppendorf tube: DNA extracted from normal corn with an amplified fragment of the zein gene |
| | • 1 C3 Eppendorf tube: DNA extracted from normal corn with an amplified fragment of the 35S gene |
| | • 1 D3 Eppendorf tube: DNA extracted from normal corn with an amplified fragment of the NOS gene |
| Test-tube rack group 4 (store between 2-8 °C) | • 1 A4 Eppendorf tube: DNA extracted from transgenic corn with an amplified fragment of the lectin gene |
| | • 1 B4 Eppendorf tube: DNA extracted from transgenic corn with an amplified fragment of the zein gene |
| | • 1 C4 Eppendorf tube: DNA extracted from transgenic corn with an amplified fragment of the 35S gene |
| | • 1 D4 Eppendorf tube: DNA extracted from transgenic corn with an amplified fragment of the NOS gene |

### MICROBIAL BIOTECHNOLOGY

| MATERIALS | COMPONENTS PER GROUP |
|---|---|
| Sterile Pasteur Pipettes | • 3 pipettes for microorganisms (A, B and C): amylase test |
| | • 3 pipettes for microorganisms (A, B and C): protease test |
| Pasteur Pipettes for reagents | • 1 AMY pipette |
| Safety tube (store between 2-8 °C) | 3 vials of microorganisms |
| | • Vial A: *Bacillus subtilis* |
| | • Vial B: *Micrococcus luteus* |
| | • Vial C: Negative Control |
| Test-tube rack (store between 2-8 °C) | • 3 AMY tubes (A, B, and C): amylase test |
| | • 3 PRO tubes (A, B, and C): protease test |
| Test-tube rack (store at room temperature) | • 1 AMY Eppendorf tube: lodinated reagent |

### BIOPRODUCTION OF CELLULASES AND β-GALACTOSIDASE

| MATERIALS | COMPONENTS PER GROUP |
|---|---|
| Sterile Pasteur Pipettes | • 2 pipettes for the microorganism (A) |
| | • 2 pipettes for the microorganism (B) |
| Pasteur Pipettes for reagents | • 1 CEL 1 pipette |
| | • 1 CEL 2 pipette |
| Safety tube (store between 2-8 °C) | 2 vials of microorganisms |
| | • Vial A: *Bacillus subtilis* |
| | • Vial B: *Micrococcus luteus* |
| Petri dishes (store between 2-8 °C) | • 2 CEL Petri dishes (skin diseases) (A and B): Cellulase test |
| Waste tube | • 1 tube for disposing of the reagents from the cellulase test |
| Test-tube rack | • 2 CEL tubes (A and B): Cellulase test |
| (store between 2-8 °C) | • 2 BG tubes (A and B): b-galactosidase test |
| Test-tube rack (store at room temperature) | • 1 CEL 1 tube: developing solution for the cellulase test |
| | • 1 CEL 2 tube: decolourizing solution for the cellulase test |

### BACTERIAL DIFFERENTIATION

| MATERIALS | COMPONENTS PER GROUP |
|---|---|
| Sterile Pasteur Pipettes | • 1 pipette for the microorganism (A) |
| | • 1 pipette for the microorganism (B) |
| | • 1 pipette for the microorganism (C) |
| | • 1 pipette for the microorganism (D) |
| Pasteur Pipettes for reagents | • 3 pipettes (IND, VP1 and VP2) |
| Sterile inoculation loops | • 2 inoculation loops for the microorganism (A) |
| | • 2 inoculation loops for the microorganism (B) |
| | • 2 inoculation loops for the microorganism (C) |
| | • 2 inoculation loops for the microorganism (D) |
| Safety tube (store between 2-8 °C) | 4 vials of microorganisms |
| | • Vial A: *Citrobacter amalonaticus* |
| | • Vial B: *Bacillus subtilis* |
| | • Vial C: *Citrobacter freundii* |
| | • Vial D: *Micrococcus luteus* |
| Test-tube rack (store between 2-8 °C | • 4 CIT tubes (A, B, C and D): citrate test |
| | • 4 IND tubes (A, B, C and D): indol test |
| | • 4 VP tubes (A, B, C and D): Voges-Proskauer test |
| | • 4 SU tubes (A, B, C and D): urease and H2S tests |
| Test-tube rack (store between 2-8 °C) | • 1 IND Eppendorf tube |
| | • 1 VP1 Eppendorf tube |
| | • 1 VP2 Eppendorf tube |

### OXYGEN TOLERANCE

| MATERIALS | COMPONENTS PER GROUP |
|---|---|
| Sterile Pasteur Pipettes | • 1 pipette (A) |
| Pasteur Pipettes for reagents | 1 VAS pipette for the oxidation/fermentation test |
| Sterile inoculation loops | • 2 inoculation loops for the oxygen relationship test (A and B) |
| | • 4 inoculation loops for the metabolism test (A and B) |
| Safety tube (store between 2-8 °C) | 2 vials of microorganisms |
| | • Vial A: *Micrococcus luteus* |
| | • Vial B: *Citrobacter freundii* |
| Petri dishes (store between 2-8 °C) | • 2 Petri dishes: aerobiosis |
| | • 2 Petri dishes: anaerobiosis |
| Test-tube rack (store between 2-8 °C | • 2 RO tubes (A and B): oxygen relationship test |
| | • 2 MC tubes (A and B): oxidation test |
| | • 2 MS tubes (A and B): fermentation test |
| | • 1 VAS Eppendorf tube |

### MOBILITY AND CHEMOTAXIS.

| MATERIALS | COMPONENTS PER GROUP |
|---|---|
| Sterile Pasteur Pipettes | • 2 pipettes (A and B) for the live culture |
| | • 2 pipettes (A and B) for mobility |
| | • 2 pipettes (A and B) for chemotaxis |
| Sterile inoculation loops | • 2 inoculation loops for mobility |
| Safety tube | 2 vials of microorganisms |
| (store between 2-8 °C) | • Vial A: *Micrococcus luteus* |
| | • Vial B: *Citrobacter freundii* |
| Test-tube rack | • 2 M tubes (A and B): mobility test |
| (store between 2-8 °C | • 2 Q tubes (A and B): chemotaxis test |
| | • 1 R Eppendorf tube: repellent substance |
| COMMON MATERIAL (included in each group box 1) | COMPONENTS PER KIT |
| Slide storage tray | • 4 slides for the live culture (one for each group) |
| Cover glasses | • 8 cover glasses (two for each group) |

## Claims

1. Portable biotechnology kit for Microbial Biotechnology or Molecular Biology practical lessons, comprising at least one group box containing all the material necessary for carrying out a Microbial or Molecular Biotechnology practical lesson, comprising the following elements:
• At least one test-tube rack, of disposable material, for holding tubes, vials or Eppendorf tubes with samples, culture mediums or reagents.
• At least one pipette.
• At least one of the following elements: a micro vial, an Eppendorf tube, an agarose gel, an electrophoresis clamp, absorbent cellulose paper for cleaning the electrophoresis clamp, a small square of blotting paper in order to load an agarose gel, a sterile inoculation loop, a safety tube
the portable biotechnology kit **characterised in that** the group box is made from a flat sheet of cardboard that can be folded into a closed container in which to transport and house all said elements, and which can be opened to form a flat surface that can be used as a work surface on which to carry out the practical lesson, the box having quick fastening means for it to be attached to a support surface when unfolded.

2. Portable biotechnology kit, according to claim 1, **characterised in that** the quick fastening means comprises an adhesive.

3. Portable biotechnology kit according to claim 1, **characterised in that** the flat cardboard sheet comprises folding lines in order to make it easy to be folded into a closed container that can house the necessary material, additionally comprising a lid that closes the container that has been formed and keeps it in place without the need to use adhesives or other fastening elements.

4. Portable biotechnology kit according to claim 1, **characterised in that** it comprises four group boxes and one cardboard box with a lid in order to house the four group boxes, as well as at least one instruction manual on how to carrying out the practical.

5. Portable biotechnology kit according to claim 1, for Molecular Biology practical lessons, namely, those of "Bacterial DNA Extraction", "Diagnosis of Maternal Kinship", "Screenings for Transgenics" and "Identification of Meat Species of Interest in Nutrition", **characterised in that** it comprises:
• At least one Teacher's Manual and one Student Manual, or otherwise an interactive CD-ROM.
• At least one test-tube rack.
• At least one group box with all the material necessary for carrying out a Microbial or Molecular Biotechnology practical lesson.
• At least one micro vial and one safety tube for microorganisms in the case of the "Bacterial DNA Extraction" kit.
• At least two Eppendorf tubes with lysis solutions in the case of the "Bacterial DNA Extraction"kit and at least four Eppendorf tubes containing DNA and a solution for DNA staining, in the other Molecular Biology kits.
• At least one sterile Pasteur pipette for inoculating microorganisms in the case of the "Bacterial DNA Extraction" kit.
• At least one non-sterile Pasteur pipette for the addition of reagents in any of the kits.
• At least one Falcon tube in any of the kits.
• At least one electrophoresis clamp for DNA loading.
• At least one absorbent cellulose paper to clean the clamps with.
• At least 4 small squares of blotting paper for gel loading.
• At least 1 agarose gel with prepared wells.

6. A portable biotechnology kit according to claim 1, for Microbial Biotechnology practical lessons, namely, those of "Bioproduction of Amylases and Proteases", "Bioproduction of Cellulases and β-Galactosidase", "Food Biotechnology", "Bacterial Differentiation", "Oxygen Tolerance" and "Mobility and Chemotaxis" **characterised in that** it comprises:
• At least one Teacher's Manual and one Student Manual, or otherwise an interactive CD-ROM.
• At least one test-tube rack.
• At least one group box with all the material necessary for carrying out a Microbial or Molecular Biotechnology practical lesson At least 2 micro vials and one safety tube for the microorganisms.
• At least one Eppendorf tube with reagents.
• At least 2 sterile Pasteur Pipettes for inoculating microorganisms and/or at least 2 sterile inoculation loops.
• At least one non-sterile Pasteur pipette for the addition of reagents.
• At least 4 11x70 tubes with culture medium.
• At least 2 Petri dishes in the kits of: "Bioproduction of Cellulases and β-Galactosidase", "Oxygen Tolerance" and "Mobility and Chemotaxis".
• At least 1 Falcon tube in the "Bioproduction of Cellulases and b-Galactosidase"and "Food Biotechnology" kits.
• At least 1 slide and 2 cover glasses for the "Mobility and Chemotaxis" kit.

## Patentansprüche

1. Tragbares Biotechnologie-Kit für praktischen Unterricht in mikrobieller Biotechnologie oder Molekularbiologie, umfassend wenigstens eine Gruppenschachtel, die das gesamte Material enthält, das zum Durchführen eines praktischen Unterrichts in mikrobieller oder molekularer Biotechnologie erforderlich ist, umfassend die folgenden Elemente:
• wenigstens ein Reagenzglasgestell, aus Einwegmaterial, zum Aufbewahren von Röhrchen, Ampullen oder Eppendorf-Röhrchen mit Proben, Kulturmedien oder Reagenzien
• wenigstens eine Pipette
• wenigstens eines der folgenden Elemente: eine Mikroampulle, ein Eppendorf-Röhrchen, ein Agarose-Gel, eine Elektrophorese-Klemme, absorbierendes Cellulosepapier zum Reinigen der Elektrophorese-Klemme, ein kleines Quadrat aus Löschpapier, um ein Agarose-Gel zu beladen, eine sterile Impföse, ein Sicherheitsröhrchen
wobei das tragbare Biotechnologie-Kit **dadurch gekennzeichnet ist, dass** die Gruppenschachtel aus einem flachen Bogen aus Pappe gebildet ist, der zu einem geschlossenen Behälter gefaltet werden kann, in dem alle besagten Elemente transportiert und untergebracht werden sollen, und der geöffnet werden kann unter Bildung einer flachen Oberfläche, welche als Arbeitsoberfläche verwendet werden kann, auf der der praktische Unterricht durchgeführt werden soll, wobei die Schachtel ein Schnellbefestigungsmittel aufweist, mit dem sie auf einer Trägeroberfläche befestigt werden soll, wenn sie entfaltet ist.

2. Tragbares Biotechnologie-Kit nach Anspruch 1, **dadurch gekennzeichnet, dass** das Schnellbefestigungsmittel einen Klebstoff umfasst.

3. Tragbares Biotechnologie-Kit nach Anspruch 1, **dadurch gekennzeichnet, dass** der flache Pappebogen Faltungslinien umfasst, so dass er leicht zu einem geschlossenen Behälter gefaltet werden kann, in dem das erforderliche Material untergebracht werden kann, zusätzlich umfassend einen Deckel, welcher den gebildeten Behälter verschließt und ihn an Ort und Stelle hält ohne das Erfordernis der Verwendung von Klebstoffen oder anderen Befestigungselementen.

4. Tragbares Biotechnologie-Kit nach Anspruch 1, **dadurch gekennzeichnet, dass** es vier Gruppenschachteln und eine Pappschachtel mit einem Deckel umfasst, um die vier Gruppenschachteln unterzubringen, sowie wenigstens ein Handbuch mit Anweisungen zur Durchführung des praktischen Unterrichts.

5. Tragbares Biotechnologie-Kit nach Anspruch 1 für praktischen Unterricht in Molekularbiologie, nämlich Unterricht in "Extraktion von bakterieller DNA", "Diagnose mütterlicher Verwandtschaft", "Screenings auf Transgene" und "Identifizierung von Fleischarten von Interesse für die Ernährung", **dadurch gekennzeichnet, dass** es umfasst:
• wenigstens ein Lehrerhandbuch und ein Schülerhandbuch oder andernfalls eine interaktive CD-ROM
• wenigstens ein Reagenzglasgestell
• wenigstens eine Gruppenschachtel mit dem gesamten Material, das zum Durchführen eines praktischen Unterrichts in mikrobieller oder molekularer Biotechnologie erforderlich ist
• wenigstens eine Mikroampulle und ein Sicherheitsröhrchen für Mikroorganismen im Fall des Kits für "Extraktion von bakterieller DNA"
• wenigstens zwei Eppendorf-Röhrchen mit Lyselösungen im Fall des Kits für "Extraktion von bakterieller DNA" und wenigstens vier Eppendorf-Röhrchen, die DNA und eine Lösung zur DNA-Anfärbung enthalten, in den anderen Molekularbiologie-Kits
• wenigstens eine sterile Pasteur-Pipette zum Einimpfen von Mikroorganismen im Fall des Kits für "Extraktion von bakterieller DNA"
• wenigstens eine nicht sterile Pasteur-Pipette für die Zugabe von Reagenzien in beliebigen der Kits
• wenigstens ein Falcon-Röhrchen in beliebigen der Kits
• wenigstens eine Elektrophorese-Klemme für die DNA-Aufgabe
• wenigstens ein absorbierendes Cellulosepapier zum Reinigen der Klemmen damit
• wenigstens 4 kleine Quadrate aus Löschpapier zur Gel-Beladung
• wenigstens 1 Agarose-Gel mit vorbereiteten Vertiefungen.

6. Tragbares Biotechnologie-Kit nach Anspruch 1 für praktischen Unterricht in mikrobieller Biotechnologie, nämlich Unterricht in "Bioproduktion von Amylasen und Proteasen", "Bioproduktion von Cellulasen und β-Galactosidase", "Lebensmittel-Biotechnologie", "bakterielle Differenzierung", "Sauerstofftoleranz" und "Mobilität und Chemotaxis", **dadurch gekennzeichnet, dass** es umfasst:
• wenigstens ein Lehrerhandbuch und ein Schülerhandbuch oder andernfalls eine interaktive CD-ROM
• wenigstens ein Reagenzglasgestell
• wenigstens eine Gruppenschachtel mit dem gesamten Material, das zum Durchführen eines praktischen Unterrichts in mikrobieller oder molekularer Biotechnologie erforderlich ist
• wenigstens 2 Mikroampullen und ein Sicherheitsröhrchen für die Mikroorganismen
• wenigstens ein Eppendorf-Röhrchen mit Reagenzien
• wenigstens 2 sterile Pasteur-Pipetten zum Einimpfen von Mikroorganismen und/oder wenigstens 2 sterile Impfösen
• wenigstens eine nicht sterile Pasteur-Pipette für die Zugabe von Reagenzien
• wenigstens 4 11 x 70-Röhrchen mit Kulturmedium
• wenigstens 2 Petrischalen in den Kits für: "Bioproduktion von Cellulasen und β-Galactosidase", "Sauerstofftoleranz" und "Mobilität und Chemotaxis"
• wenigstens 1 Falcon-Röhrchen in den "Bioproduktion von Cellulasen und β-Galactosidase" und "Lebensmittel-Biotechnologie"-Kits
• wenigstens 1 Objektträger und 2 Deckgläser für den "Mobilität und Chemotaxis"-Kit.

## Revendications

1. Kit biotechnologique portable pour des leçons pratiques de biotechnologie ou de biologie moléculaire comprenant au moins une boîte de regroupement contenant le matériel nécessaire pour mettre en oeuvre une leçon pratique de biotechnologie microbienne ou moléculaire comprenant les éléments suivants :
- au moins un portoir de tubes à essai en un matériau jetable pour maintenir des tubes, des vials ou des tubes d'Eppendorf avec des échantillons, des milieux de culture ou des réactifs,
- au moins une pipette,
- au moins l'un des éléments suivants : un micro vial, un tube d'Eppendorf, un gel d'agarose, une pince pour électrophorèse, un papier cellulosique absorbant pour nettoyer la pince pour électrophorèse, un petit carré de papier buvard pour charger un gel d'agarose, une anse d'inoculation stérile, un tube de sécurité,
ce kit biotechnologique portable étant **caractérisé en ce que** la boîte de regroupement est constituée d'une feuille plate de carton qui peut être pliée de manière à former un réceptacle fermé pour le transport et la réception de tous ces éléments, et qui peut être ouverte de façon à former une surface plane pouvant être utilisée en tant que surface de travail permettant la mise en oeuvre de la leçon pratique, cette boîte ayant des moyens d'attache rapide permettant sa fixation à une surface support lorsqu'elle n'est pas pliée.

2. Kit biotechnologique portable selon la revendication 1,
**caractérisé en ce que**
les moyens d'attache rapide comprennent un adhésif.

3. Kit biotechnologique portable selon la revendication 1,
**caractérisé en ce que**
la feuille de carton plane comprend des lignes de pliage pour faciliter son repliement sous la forme d'un réceptacle fermé pouvant recevoir le matériel nécessaire, et comprenant en outre un couvercle refermant le réceptacle ayant ainsi été formé et le maintenant en place sans nécessiter d'utiliser des adhésifs ou d'autres éléments de fixation.

4. Kit biotechnologique portable selon la revendication 1,
**caractérisé en ce qu'**
il comprend quatre boîtes de regroupement et une boîte en carton avec un couvercle pour recevoir les quatre boîtes de regroupement ainsi qu'au moins un manuel d'instruction précisant comment doivent être mis en oeuvre les travaux pratiques.

5. Kit biotechnologique portable selon la revendication 1, pour des leçons pratiques de biologie moléculaire à savoir celles consistant en « l'extraction d'ADN bactérien », « le diagnostic de parenté de matière », « le criblage de transgéniques » et « l'identification d'espèces de nourriture intéressantes dans le cadre de la nutrition »,
**caractérisé en ce qu'**il renferme :
- au moins un manuel d'enseignant et un manuel d'étudiant ou un CD-ROM interactif,
- au moins un portoir de tubes à essai,
- au moins une boîte de regroupement avec tout le matériel nécessaire pour la mise en oeuvre de la leçon pratique de biotechnologie microbienne ou moléculaire,
- au moins un micro vial et un tube de sûreté pour des microorganismes dans le cas d'un kit « d'extraction d'ADN bactérien »
- au moins deux tubes d'Eppendorf avec des solutions de lyses dans le cas d'un kit « d'extraction d'ADN bactérien » et au moins quatre tubes d'Eppendorf renfermant de l'ADN et une solution pour colorer l'ADN dans les autres kits de biologie moléculaire,
- au moins une pipette de Pasteur stérile pour inoculer des microorganismes dans le cas d'un kit « d'extraction d'ADN bactérien »,
- au moins une pipette de Pasteur non stérile pour l'addition de réactifs dans chacun des kits,
- au moins un tube de Falcon dans chacun des kits,
- au moins une pince pour électrophorèse pour charger l'ADN,
- au moins un papier cellulosique absorbant pour nettoyer les pinces,
- au moins 4 petits carrés de papier buvard pour charger le gel,
- au moins 1 gel d'agarose avec des puits préparés.

6. Kit biotechnologique portable selon la revendication 1, pour des leçons pratiques de biotechnologie microbienne à savoir, concernant la « bioproduction d'amylases et de protéases », la « bioproduction de cellulases et β-galactosidase », la « biotechnologie nutritionnelle », la « différentiation bactérienne », la « tolérance à l'oxygène » et « la mobilité et le chimiotactisme »,
**caractérisé en ce qu'**il comporte :
- au moins un manuel d'enseignant et un manuel d'étudiant ou un CD-ROM interactif,
- au moins un portoir de tubes à essai,
- au moins une boîte de regroupement avec tout le matériel nécessaire pour la mise en oeuvre d'une leçon pratique de biotechnologie microbienne ou moléculaire,
- au moins 2 micro vials et un tube de sécurité pour les microorganismes,
- au moins un tube d'Eppendorf avec des réactifs,
- au moins 2 pipettes de Pasteur stériles pour inoculer des microorganismes et/ou au moins 2 anses d'inoculation stériles,
- au moins une pipette de Pasteur non stérile pour l'addition de réactifs,
- au moins 4 tubes 11x70 avec un milieu de culture,
- au moins 2 boîtes de Petri dans les kits pour la « bioproduction de cellulases et β-galactosidase », de « tolérance à l'oxygène » et de « mobilité et chimiotaxis »,
- au moins 1 tube de Falcon pour les kits de « bioproduction de cellulases et β-galactosidase » et de « biotechnologie nutritionnelle »,
- au moins 1 verre glissant et 2 verres de recouvrement dans le kit de « mobilité et chimiotactisme ».
